# EUROPEAN PATENT APPLICATION

(11) **EP 4 756 115 A1**
(43) Date of publication of application: **10.06.2026**
(21) Application number: 24903846.4
(22) Date of filing: 03.06.2024
(51) Int. Cl.: D06F 58/12, D06F 58/20

(54) **CLOTHING PROCESSING DEVICE**

(30) Priority: 14.12.2023 KR 20230181997
(71) Applicant: LG Electronics Inc., Yeongdeungpo-gu Seoul 07336 (KR)
(72) Inventor: KIM, Sangjin, Seoul 08592 (KR); KIM, Sunghoon, Seoul 08592 (KR)
(74) Representative: Vossius & Partner Patentanwälte Rechtsanwälte mbB
(86) International application number: PCT/KR2024/007550
(87) International publication number: WO 2025/127274

(57) **Abstract**

Disclosed herein is a laundry treating apparatus may include a cabinet, an inner case disposed in the cabinet to provide an accommodation space, a machine chamber arranged to communicate with the inner case to supply at least one of steam or air to the accommodation space, and a moving hanger arranged to shake the laundry. The moving hanger may include a power transmitter including a hollow portion disposed in the accommodation space in a height direction of the inner case and having at least a portion of one surface open, a drive part coupled to the power transmitter to cause the power transmitter to rotate or translate, and a hanging part provided on both side surfaces of the power transmitter, wherein the power transmitter may include a reinforcing rib disposed to connect the both side surfaces of the power transmitter to divide the hollow portion into upper and lower portions.

## Description

### [Technical Field]

The present disclosure relates to a laundry treating apparatus. More specifically, it relates to a laundry treating apparatus for performing a refresh procedure in which at least one of air and steam is supplied to laundry to perform at least one of deodorization, de-wrinkling, and sanitization of the laundry.

### [Background]

A laundry treating apparatus is an apparatus developed to wash and dry laundry, and to remove wrinkles from laundry, both at home and in laundry facilities. The concept of a laundry treating apparatus includes a washing machine to wash laundry, a dryer to dry laundry, a washer/dryer that has both washing and drying functions, a laundry care machine to refresh laundry, and a steamer to remove wrinkles from laundry.

Recently, a laundry treating apparatus corresponding to a laundry care machine has emerged that enables laundry to be kept fresh and clean without soaking it in water and washing it with detergent.

Among such conventional laundry treating apparatuses is a laundry treating apparatus that performs a refreshing procedure by supplying either hot air or steam to the laundry to deodorize the laundry, dry the laundry, and remove wrinkles from the laundry.

A laundry treating apparatus capable of performing the refreshing procedure may be configured to vibrate the laundry while supplying hot air or steam to the laundry, in order to shake off any fine dust or debris stuck to the laundry (see Korean Patent No. 10-1285890).

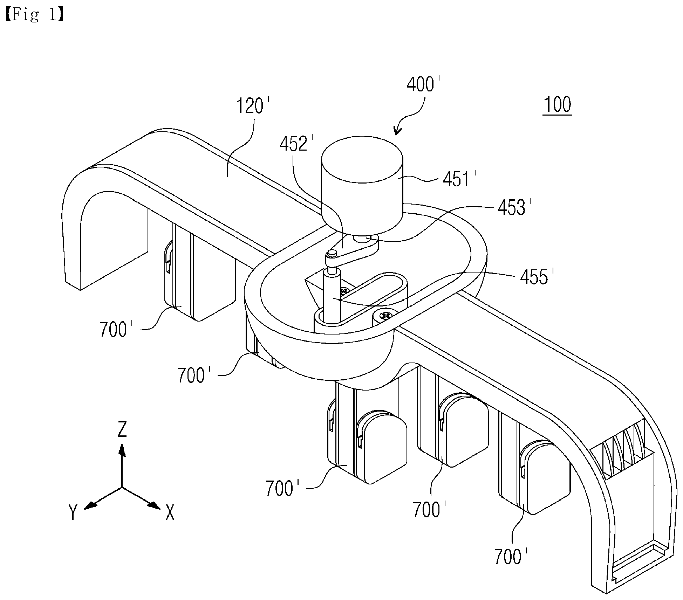

FIG. 1 illustrates a structure for vibrating laundry in a conventional laundry treating apparatus.

A moving hanger 100 of a conventional laundry treating apparatus includes a support bar 120' disposed in a accommodation space in which the laundry is mounted, a hanging part 700' extending downward from the support bar 120' to allow the laundry to be mounted thereon, and a drive part 400' configured to vibrate the support bar 120'.

The drive part 400' may include a motor 451' fixed to an upper portion of the support bar 120' to rotate a rotation shaft 453', and may include an eccentric shaft 455' coupled to the rotation shaft 453' to rotate in a trajectory greater than a radius of rotation of the rotation shaft 453'.

A reciprocation guide to accommodate the eccentric shaft to receive transmitted power is provided in the center of the support bar 120'. The eccentric shaft 455' may be arranged to move according to the rotation of the rotation shift 453' while coupled to the reciprocation guide 400' and to move the reciprocation guide 400' from side to side in a reciprocating manner.

The eccentric shaft 455' may be coupled to an end of a connecting shaft 452' coupled to an end of the rotation shaft 453' and arranged to rotate.

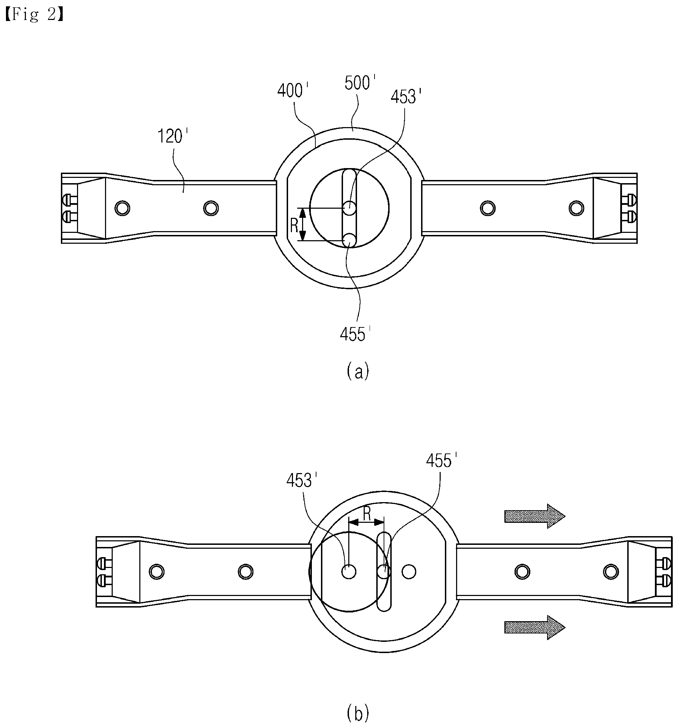

FIG. 2 illustrates a structure in which the support bar moves from side to side.

The reciprocation guide 400' may include a slit formed in the thickness direction of the support bar 120' to accommodate the eccentric shaft 455'.

FIG. 2 illustrates a structure in which the support bar moves from side to side.

The reciprocation guide 400' may include a slit formed in the thickness direction of the support bar 120' to accommodate the eccentric shaft 455'.

Referring to FIG. 2-(a), the eccentric shaft 455' may be inserted into the slit and arranged to rotate in an arc trajectory with a radius R, which is a distance from the rotation shift 453'.

The support bar 120' may be movably fixed in the laundry treating apparatus to move only from side to side and not to move back and forth.

Referring to FIG. 2-(b), when the eccentric shaft 455' is rotated 90 degrees to the right, the slit may move with the eccentric shaft 455' by R to the right, due to the eccentric shaft 455' being moved by R to the right. As a result, the support bar 120' is moved to the right.

In this way, when the eccentric shaft 455' is rotated 180 degrees to the left, the slit will move to the left, and the support bar 120' will also move to the left.

When the rotation shaft 453' rotates once, the support bar 120' is allowed to make one reciprocating movement to the left and right. When the rotation shaft 453' rotates continuously, the support bar 120' makes several reciprocating movements to the left and right.

As a result, the laundry mounted on the hanging part 700' extending from the support bar 120' may be vibrated from side to side such that foreign matter or dirt may be separated.

However, in such a conventional laundry treating apparatus, as the support bar 120' makes reciprocating movements to the left and right, the entire laundry treating apparatus vibrates strongly due to a sudden inertia force whenever the support bar 120' changes the direction of movement.

In particular, as the amount or weight of the laundry mounted on the hanging part 700' increases, the amount of vibration will increase, and the stability of the laundry treating apparatus may not be guaranteed.

In addition, in the conventional laundry treating apparatus, as the vibration of the laundry treating apparatus increases, the noise of the laundry treating apparatus also increases. Thus, the noise is emitted every time the laundry treating apparatus is operated, and the use of the laundry treating apparatus is limited at night, etc.

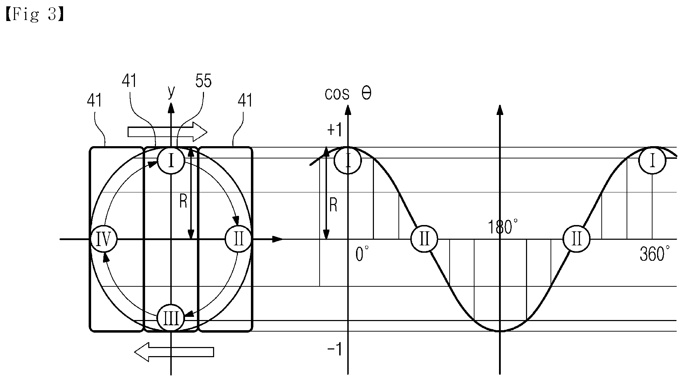

FIG. 3 illustrates the loss of output power of the drive part of the conventional laundry treating apparatus.

In addition, in the conventional laundry treating apparatus, even if the drive part 5 can apply sufficient output power, the maximum output power of the drive part 5 may be unavailable because the vibration generated by the reciprocating movement of the support bar 2 exceeds a reference value.

In addition, a moving hanger that reciprocates from side to side, as in the conventional laundry treating apparatus, cannot receive the full rotational power of the drive part 3.

Specifically, the slit 41 in the support bar 2 of the moving hanger may transmit the force of the eccentric shaft 55 rotating leftward or rightward to the laundry. However, it cannot transmit the force of the eccentric shaft 55 rotating backward or forward to the laundry at all, thereby dissipating the force.

Specifically, when the eccentric shaft 55 is disposed in positions I and III, the slit 41 may be subjected to forces that move the slit 41 to the right and left.

However, when the eccentric shaft 55 moves from position I to position II, or when the eccentric shaft 55 moves from position III to position IV, the force transmitted by the eccentric shaft 55 to move the slit 41 from side to side decreases drastically.

This is because the force of the eccentric shaft 55 to move the slit 41 is only the cosine of the force in the direction of rotation of the eccentric shaft 55, which means that the power transmitted from the eccentric shaft 55 to the slit 41 is zero when the eccentric shaft 55 is at 90 and 270 degrees with respect to the center of rotation.

In other words, although the drive part 5 continuously transmits power to the eccentric shaft 55, the eccentric shaft 55 can only transmit part of the power to the slit 41, and even cannot transmit the power at all at particular points.

As a result, in the conventional laundry treating apparatus, most of the power of the drive part 5 is dissipated without being transmitted to the laundry. Accordingly, the conventional laundry treating apparatus either does not transmit sufficient vibration force to the laundry even though the drive part 3 has sufficient power to vibrate the laundry, or the drive part 3 must be driven with excessive power to transmit sufficient vibration force to the laundry.

As a result, the conventional laundry treating apparatus has a fundamental problem that the laundry does not receive sufficient vibration force from the drive part 3 to separate the foreign matter or dirt stuck to the laundry.

In addition, in a conventional laundry treating apparatus, the support bar 2 is arranged in the width direction and the hanging part 3 is fixed to the support bar 2. Therefore, the rotational force generated by the drive part 3 cannot be fully transmitted to the support bar 2.

To address this issue, a laundry treating apparatus has been invented in which the hanging part 3 on which the laundry is mounted can be shaken by a reciprocating rotary motion rather than a linear reciprocating motion (See Chinese Public Patent Gazette No. 111759148).

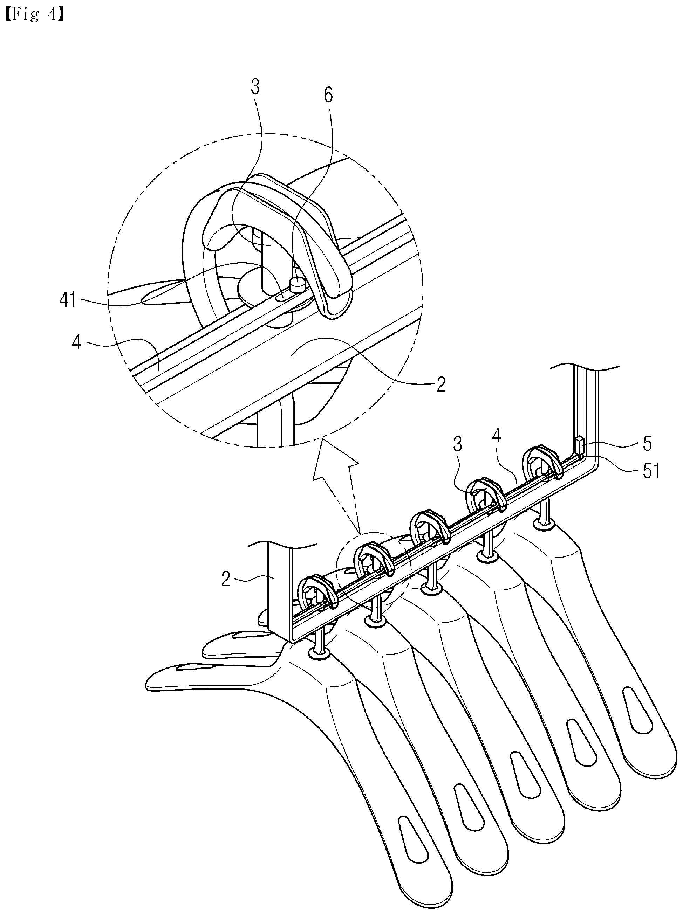

FIG. 4 illustrates a rotating structure of hanging part in the related art.

The hanging part 3 is rotatably mounted on the upper portion of the support bar 2, and is provided with a link part 5 mounted on the hanging part 3 to reciprocate to the left and right.

The reciprocation guide 4 may be coupled to an outer circumferential surface of the hanging part 3 by a fastening member 6.

The portion of the hanging part 3 to which the fastening member 6 is coupled may be spaced apart from the center of rotation of the hanging part 3.

The reciprocation guide 4 may be configured as a bar-shaped link. In moving the reciprocation guide 4 from side to side in the width direction in a reciprocating manner, the fastening member 6 may cause the reciprocation guide 4 to reciprocate.

As a result, the hanging part 3 may be rotated from side to side in a reciprocating manner, and laundry mounted on the hanging part 3 may also be rotated in a reciprocating manner.

However, in the related art, when the link part 5 is rotated in a reciprocating manner, the reciprocation guide 4 must move forward and backward to a certain extent according to the rotational trajectory of the hanging part 3. As a result, the side-to-side reciprocating movement of the reciprocation guide 4 cannot be a perfect side-to-side reciprocating movement.

In the case where multiple hanging parts 3 are arranged spaced apart from each other, and one reciprocation guide 4 is caused to reciprocate by the drive part 3, it may seem that the reciprocation guide can reciprocate the multiple hanging parts 3.

However, in order for the multiple hanging parts 3 to rotate in a reciprocating manner, the reciprocation guide 4 coupled to the hanging parts 3 must be moved forward or backward to correspond to the respective rotational trajectories of the hanging parts 3. Since one end of the reciprocation guide 4 is fixed to an end 51 of the drive part 3, the reciprocation guide 4 cannot move forward or backward as a whole to correspond to the rotational trajectories of the hanging parts 3.

In other words, the reciprocation guide 4 moves forward or backward at a certain angle around the end thereof coupled with the drive part 3. In other words, the opposite end of the reciprocation guide 4 farther away from the drive part 3 has a larger amplitude than the one end of the reciprocation guide 4.

Therefore, when the multiple hanging parts 3 are arranged side by side along the width direction of the support bar 2, it is theoretically impossible to rotate all the hanging parts 3 with the reciprocation guide 4.

Accordingly, the reciprocation guide 4 cannot rotate the multiple hanging parts 5 at once and simultaneously.

As a result, the conventional technology suffers from the fundamental problem that when the hanging part 3 includes multiple hanging parts, the multiple hanging parts 3 cannot be rotated in a reciprocating manner simultaneously or at once.

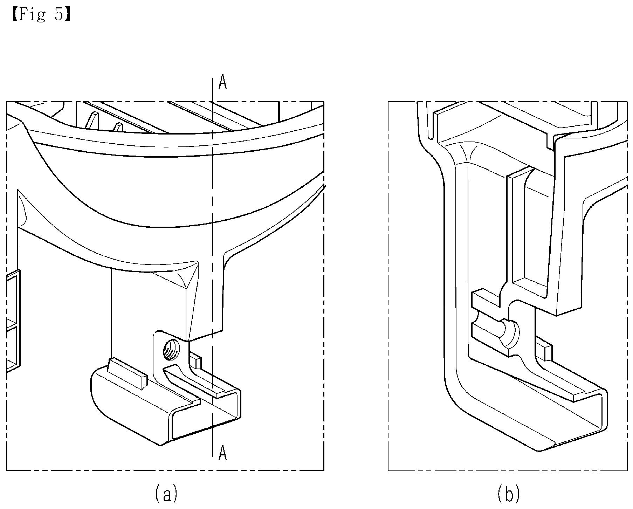

FIG. 5 is a cross-sectional view of the hanging part in a conventional laundry treating apparatus.

The hanging part is designed based on the case where there is no rotational or translational movement of the laundry. Therefore, the hanging part is designed to withstand only the load caused by the weight of the laundry.

However, as described above, in a conventional laundry treating apparatus, the laundry is subjected to a translational or rotational movement by a moving hanger, and increasing the rigidity to withstand such a load is an important issue in a laundry treating apparatus having a moving hanger.

### [Summary]

### [Technical Problem]

An object of the present disclosure is to provide a laundry treating apparatus having a moving hanger capable of withstanding a load of laundry caused by gravity.

Another object of the present disclosure is to provide a laundry treating apparatus having a moving hanger capable of withstanding a load caused by a translational motion of laundry.

Another object of the present disclosure is to provide a laundry treating apparatus having a moving hanger capable of withstanding a load caused by a rotational motion of laundry.

### [Technical Solutions]

To achieve these objects and other advantages and in accordance with the purpose of the disclosure, as embodied and broadly described herein, a laundry treating apparatus may include a cabinet, an inner case disposed in the cabinet to provide an accommodation space to place laundry, a machine chamber arranged to communicate with the inner case to supply at least one of steam or air to the accommodation space, and a moving hanger arranged to shake the laundry placed in the accommodation space, wherein the moving hanger may include a power transmitter including a hollow portion disposed in the accommodation space in a height direction of the inner case and having at least a portion of one surface open, a drive part coupled to the power transmitter to provide power to cause the power transmitter to rotate or translate, and a hanging part provided on both side surfaces of the power transmitter except for the one surface of the power transmitter to place the laundry, wherein the power transmitter may include a reinforcing rib disposed to connect the both side surfaces of the power transmitter to divide the hollow portion into upper and lower portions to enhance rigidity of the power transmitter.

In one embodiment, the reinforcing ribs may include at least one of a first reinforcing rib having one end connected to one of the side surfaces of the power transmitter and an opposite end connected to the other one of the side surfaces of the power transmitter, the one end being disposed at a higher position than the opposite end, a second reinforcing rib having one end connected to the one side surface of the power transmitter and an opposite end connected to the other side surface of the power transmitter, the one end being disposed at a lower position than the opposite end, or a third reinforcing rib having one end connected to the one side surface of the power transmitter and an opposite end connected to the other side surface of the power transmitter, the one end being disposed at a position corresponding to the other end.

In one embodiment, the first reinforcing rib may include a plurality of first reinforcing ribs arranged spaced apart from each other.

In one embodiment, the second reinforcing rib may include a plurality of second reinforcing ribs arranged spaced apart from each other.

In one embodiment, the third reinforcing rib may include a plurality of third reinforcing ribs arranged spaced apart from each other.

In one embodiment, the second reinforcing rib may be arranged on at least one of top and bottom of at least one of the first reinforcing ribs.

In one embodiment, the plurality of second reinforcing ribs and the plurality of first reinforcing ribs may be alternately disposed in a longitudinal direction of the power transmitter.

In one embodiment, the third reinforcing rib may be arranged on at least one of top and bottom of at least one of the first reinforcing ribs.

In one embodiment, the plurality of third reinforcing ribs and the plurality of first reinforcing ribs are alternately disposed in a longitudinal direction of the power transmitter.

In one embodiment, the third reinforcing rib may be arranged on at least one of top and bottom of at least one of the second reinforcing ribs.

In one embodiment, the plurality of third reinforcing ribs and the plurality of second reinforcing ribs may be alternately disposed in a longitudinal direction of the power transmitter.

In one embodiment, the hollow portion may include a top hollow portion disposed above the reinforcing rib, and a bottom hollow portion disposed below the reinforcing rib.

In one embodiment, the bottom hollow portion has a narrower width than the top hollow portion.

In one embodiment, the bottom hollow portion has a wider width than the top hollow portion.

In one embodiment, a width of the bottom hollow portion may correspond to a width of the top hollow portion.

In one embodiment, the reinforcing rib may include a plurality of reinforcing ribs, wherein the hollow portion may include a first hollow portion disposed above a top reinforcing rib among the reinforcing ribs, a second hollow portion disposed below a bottom reinforcing rib among the reinforcing ribs, and a third hollow portion disposed between the first hollow portion and the second hollow portion.

In one embodiment, the second hollow portion may have a narrowest width.

In one embodiment, the first hollow portion may have a narrowest width.

In one embodiment, a width of the first hollow portion and a width of the second hollow portion may be less than a width of the third hollow portion.

In one embodiment, the power transmitter may include a plurality of power transmitters arranged spaced apart from each other.

In one embodiment, the machine chamber may be disposed under the inner case.

In one embodiment, the power transmitter may include a coupling portion coupled to the drive part, and a body protruding downward from the coupling portion and containing the hollow portion, wherein the hanging part may be coupled to both side surfaces of the body.

### [Advantageous Effects]

According to the present disclosure, the load of laundry caused by gravity may be withstood.

According to the present disclosure, the load caused by the translational motion of the laundry may be withstood.

According to the present disclosure, the load caused by the rotational motion of the laundry may be withstood.

### [Brief Description of the Drawings]

FIG. 1 illustrates a moving hanger of a conventional laundry treating apparatus;
FIG. 2 illustrates the principle of operation of the moving hanger of FIG. 1;
FIG. 3 illustrates a power loss of the moving hanger of FIG. 1;
FIG. 4 illustrates a conventional moving hanger in the related art;
FIG. 5 illustrates a conventional hanging part in the related art;
FIG. 6 illustrates a structure of a laundry treating apparatus according to the present disclosure;
FIG. 7 illustrates a moving hanger of the laundry treating apparatus according to the present disclosure;
FIG. 8 illustrates the operation of the moving hanger according to the present disclosure;
FIG. 9 illustrates an embodiment of the moving hanger according to the present disclosure; and
FIGS. 10 to 14 illustrate an embodiment of a reinforcing rib according to the present disclosure.

### [Best Mode]

Hereinafter, embodiments disclosed herein will be described in detail with reference to the accompanying drawings. In the present disclosure, the same or similar reference numerals are assigned to the same or similar elements in different embodiments, and each of them will be described only once. As used herein, the singular forms "a", "an" and "the" include plural forms unless the context clearly indicates otherwise. In addition, in describing the embodiments disclosed herein, when it is determined that the detailed description of the related known technology may obscure the gist of the embodiments, the detailed description thereof will be omitted. In addition, it should be noted that the accompanying drawings are merely intended to provide a further understanding of the embodiments disclosed in the present disclosure and are not to be construed as limiting the ideas disclosed herein.

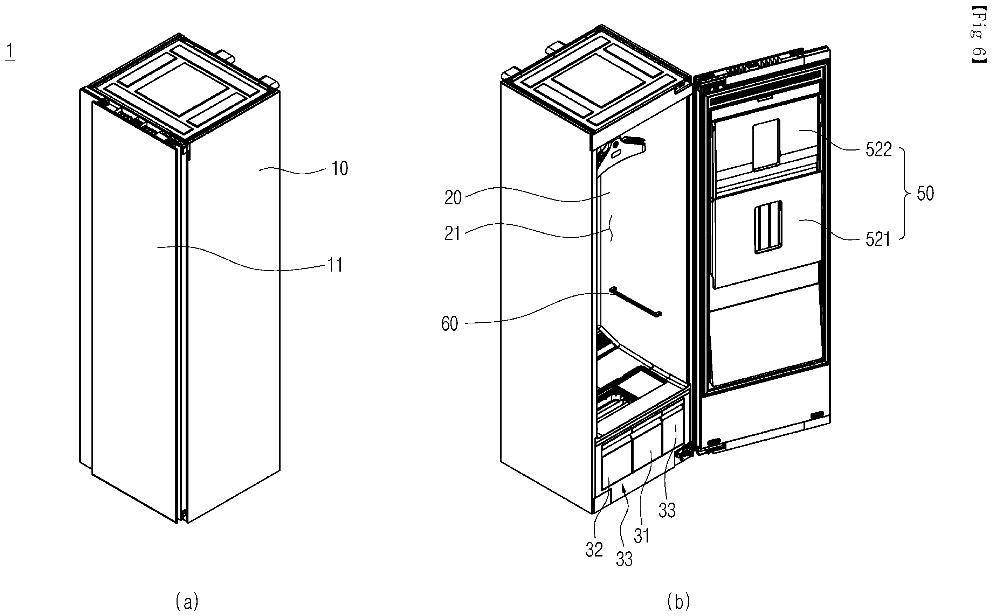

FIG. 6 illustrates the exterior of a laundry treating apparatus 1 according to the present disclosure.

Referring to FIG. 6-(a), the laundry treating apparatus according to the present disclosure may include a cabinet 10 defining the exterior, and a door 11 rotatably coupled to the cabinet 10.

Referring to FIG. 6-(b), an inner case 20 may be arranged inside the cabinet 10, the inner case 20 having an accommodation space 21 for accommodating laundry. The inner case 20 may be provided with an opening at a front side through which the laundry is introduced and withdrawn, the opening may be shielded by the door 11.

The inner case 20 may be formed of a plastic resin, such as a reinforced plastic resin that is not deformed by air at a temperature higher than room temperature or by heated air (hereinafter, hot air), steam, or moisture.

The inner case 20 may be longer in height than in width. Thereby, the laundry may be received in the accommodation space 21 without being folded or creased.

The laundry treating apparatus 1 may include a clothing hanger 900 for placing laundry in the accommodation space 21 of the inner case 20.

The clothing hanger 900 may be disposed on an top surface of the inner case 20 and may be seated in the hanging part 700 for mounting the laundry. The clothing hanger 900 may be removably disposed on the hanging part 700.

Once the laundry is mounted on the hanging part 700, the laundry may be disposed suspended in the air within the accommodation space 21.

The laundry treating apparatus of the present disclosure may further include a pressing part 50 coupled to the inner surface of the door 11 to hold the laundry.

The pressing part 50 may include a pressing surface 522 disposed on the inner surface of the door 11, and a pressing panel 521 rotatably coupled to the pressing surface 522 to press laundry disposed on the pressing surface 522. The pressing part 50 may primarily press elongated laundry, such as pants, to remove wrinkles and create an intended crease.

The according to the present disclosure may include a machine chamber 30 in which various devices are installed to supply at least one of hot air or steam to the accommodation space 21, or to purify or dehumidify the external air in the cabinet 10.

The machine chamber 30 may be arranged separately or compartmentally from the inner case 20, but may be arranged to communicate with the inner case 20.

The machine chamber 30 may be disposed in a lower portion of the inner case 20. Thus, when hot air and steam with a low specific gravity are supplied to the inner case 20, the hot air and steam may be naturally supplied to the laundry.

The machine chamber 30 may include a circulation duct for circulating air inside the inner case 20, and a plurality of heat exchangers disposed on the circulation duct to cool and condense the air, and to heat the air.

The mechanical room 30 may be equipped with a heat pump system including a compressor connected to the plurality of heat exchangers to compress a refrigerant that cools or heats the air.

Furthermore, the machine chamber 30 may be equipped with a steam supplier configured to supply steam to the inner case 20.

The steam supplier may be configured to heat water to generate the steam. Thus, the laundry accommodated in the inner case may be exposed to hot air and steam to deodorize, sanitize, and de-wrinkle the laundry.

The front of the machine chamber 30 may include a water tank 31 to supply water to generate the steam, and a drain tank 32 for collecting condensed water from the circulation duct.

The water tank 31 and drain tank 32 may be removably provided in the front of the machine chamber 30. Thereby, even if the laundry treating apparatus according to the present disclosure is not disposed near a water source or a sewer, the user may remove and carry the water tank 31 and drain tank 32 whenever necessary.

The water tank 31 and the drain tank 32 may be disposed side by side along a width direction of the machine chamber 30.

The machine chamber 30 may further include a drawer 33 to accommodate items necessary to manage the laundry. The drawer 33 may be arranged in the machine chamber 30 to be withdrawn, and may have space inside to accommodate items such as an iron.

A seating base 60 on which a separate shelf may be seated may be provided in the inner case 20. The seating base 60 may protrude from both sides of the inner case 20 at the same height.

The seating base 60 may be provided with a light emitter to emit light into the inner case 20. The light emitter may be arranged to emit light toward an inner side surface of the inner case 20 to prevent glare.

The laundry treating apparatus according to the present disclosure may further include a moving hanger 100 to shake the clothing hanger 900 to remove foreign matter, dust, and the like from the laundry placed on the clothing hanger 900.

The moving hanger 100 may be operated when at least one of hot air and steam is supplied to the laundry, thereby causing foreign matter stuck to the laundry to be separated by hot air or steam.

The moving hanger 100 may cause the clothing hanger 900 to reciprocate and vibrate.

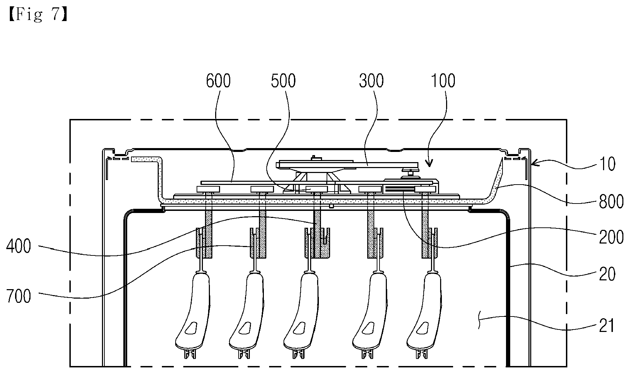

FIG. 7 shows the structure of the upper portion of the inner case.

The moving hanger 100 of the laundry treating apparatus according to the present disclosure may include a power transmitter 400 disposed in the upper portion of the inner case 10 to shake the clothing hanger 900.

The power transmitter 400 may include a hollow portion open on one side. Further details of the structure will be described later.

A hanging part 700 on which the clothing hanger 900 may be seated or mounted may be provided to a lower portion of the power transmitter 400. The hanging part may be coupled to both side surfaces of the power transmitter except the one surface of the power transmitter. Details of the structure will be described later.

Thus, when the power transmitter 400 is moved, the hanging part 700 may be moved, and the clothing hanger 900 mounted on the hanging part 700 may be shaken to shake the laundry.

The power transmitter 400 may include a plurality of power transmitters, and the hanging part 700 coupled to the power transmitter 400 may include a plurality of hanging parts. Thus, as many pieces of laundry as the number of the power transmitters 400 may be placed and refreshed inside the inner case 20.

The moving hanger 100 may further include a drive part 200 configured to provide power to move the power transmitter 400.

The drive part 200 may be arranged to be exposed to the inside of the inner case 20 as long as it is capable of transmitting power to the power transmitter 400. However, since the drive part 200 is configured to operate with electrical energy supplied, it may be prevented from being exposed to steam or hot air.

Accordingly, the drive part 200 may be disposed between the top surface of the inner case 20 and the cabinet 10 so as not to be exposed to the accommodation space 21.

The power transmitter 400 may receive power from the drive part 200 through the inner case 20 and transmit the same to the hanging part 700.

The power transmitter 400 may be arranged through the top surface of the inner case 20 in a penetrating manner to extend into the accommodation space 21. The lower end of the power transmitter 400 may be exposed to the accommodation space 21, and the upper end of the power transmitter 400 may be exposed above the inner case 20.

The inner case 200 or the support portion 800 may further include a sealing member to seal the area penetrated by the power transmitter 400. The sealing member may include a support bearing that rotatably supports the power transmitter 400 by being coupled to a hole that allows the power transmitter 400 to be arranged therethrough in the inner case 200 and the support part 800.

Thus, air and steam supplied into the inner case 200 may be blocked from leaking to a space above the top surface of the inner case 200 or above the support 800.

As a result, the drive part 200 may be operated reliably without problems such as leakage, short circuit, overheating, and the like being caused in wires seated above the support 800.

The power transmitter 400 may be formed in a rod shape, a tubular shape, a plate shape, or the like that is longer in the length direction than in the thickness direction.

The top surface of the inner case 20 may support the load of the power transmitter 400 and the drive part 200. The laundry is mounted and moved on the power transmitter 400, and the load of the drive part 200 is also relatively heavy. Accordingly, to ensure stable installation of the moving hanger 100 on the top surface of the inner case 20, the laundry treating apparatus 1 may further include a support 800.

The support 800 may be disposed in the upper portion of the inner case 20, and may be coupled to and supported by the cabinet 1. The support 800 may be formed of a durable, non-deformable metal material.

the power transmitter 400 and the drive part 200 may be seated on the support 800 and disposed in the upper portion of the inner case 20. The power transmitter 400 may extend into the accommodation space 21 through the support 800.

The drive part 200 includes a motor configured to rotate the rotation shaft. The drive part 200 may be configured to move the power transmitter 400 with power to rotate the rotation shaft.

However, it may be difficult to shake the power transmitter 400 with sufficient displacement by simply rotating the rotation shaft in place.

Accordingly, the moving hanger 100 may further include a displacement generator 300 coupled to the rotation shaft rotated by the motor to generate sufficient displacement that allows the power transmitter 400 to move.

The displacement generator 300 may be connected or coupled to the drive part 200.

For example, the displacement generator 300 may be configured to transmit power from the drive part 200 to the power transmitter 400.

The displacement generator 300 may include an eccentric shaft that rotates along a trajectory that is larger than the diameter of the rotation shaft. The eccentric shaft, which may be directly coupled to the rotation shaft of the drive part 200, may be eccentrically coupled to or extend to the power shaft 240 that is rotated by the rotation shaft.

The displacement generator 300 may be in any form as long as it is capable of generating a displacement that causes the power transmitter 400 to reciprocate over a range. Details of the structure will be described later.

When the drive part 200 is operated, the power generated by the rotation shaft may generate a displacement of the displacement generator 300, and the power transmitter 400 may move according to the displacement of the displacement generator 300.

The displacement generator 300 may directly move the power transmitter 400, or may move the power transmitter 400 through an additional component.

The moving hanger 100 may be arranged to cause the power transmitter 400 to reciprocate.

Also, the first moving hanger 100 may be arranged to rotate the power transmitter 400. Specifically, the moving hanger 100 may be arranged to rotate the power transmitter 400 over a range of angles rather than causing the power transmitter 400 to reciprocate in a straight line.

The power transmitter 400 may be arranged to reciprocate clockwise or counterclockwise from a predetermined position, and the laundry mounted on the power transmitter 400 may also reciprocate clockwise or counterclockwise. The power transmitter 400 may be rotated by the moving hanger 100, but may not be moved by changing its position to the left or right.

Even when the laundry is rotated inside the inner case 20 due to the power transmitter 400, the shift of the center of gravity thereof inside the inner case 20 may be limited. Accordingly, even when the moving hanger 100 is operated, the vibration generated inside the inner case 20 may be drastically reduced, and noise generation may be minimized.

To this end, the moving hanger 100 may further include a reciprocating rotation part 500 that converts the continuous rotational energy generated by the drive part 200 or the displacement generator 300 into a reciprocating rotational motion of the power transmitter 400.

The reciprocating rotation part 500 may be configured to connect the displacement generator 300 and the power transmitter 400 to each other. The reciprocating rotation part 500 may be configured to connect the displacement generator 300 and the power transmitter 400 to each other at a higher position than the inner case 20. The reciprocating rotation part 500 may be prevented from being exposed to the accommodation space 21, thereby preventing the laundry from being damaged by the reciprocating rotation part 500.

The moving hanger 100 may be arranged to rotate only one of the plurality of power transmitters 400 in a reciprocating manner.

However, if only one of the power transmitters 400 is rotated, the laundry mounted on the rotating power transmitter may be damaged by colliding with the laundry mounted on the other power transmitters 400. Furthermore, the impact may be transmitted to the moving hanger 100 and the moving hanger 100 may be damaged.

Therefore, the moving hanger 100 may be arranged to rotate all the power transmitters 400.

The moving hanger 100 may rotate the plurality of power transmitters 400 as a whole. The moving hanger 100 may rotate the plurality of power transmitters 400 by the same angle at once. Thereby, the laundry treating apparatus of the present disclosure may prevent the power transmitters 400 from colliding with each other.

It may be advantageous in rotating all the power transmitters 400 for the power generated by the drive part 200 to be transmitted directly to the plurality of power transmitters 400.

However, in the case where the drive part 200 is configured to directly transmit power to each of the power transmitters 400, the structure connecting the drive part 200 to all the power transmitters 400 may become complicated.

In addition, when the drive part 200 includes multiple drive parts, or when the drive part 200 includes multiple components connecting all the power transmitters 400, excessive load may be imposed on the inner case 20 or the support 800. In addition, inconvenience of controlling the multiple drive parts 200 may occur.

Furthermore, when the displacement generator 300 and the reciprocating rotation part 500 are connected to transmit the power transmitted by one drive part 200 to all the power transmitters 400, respectively, the arrangement and structure of the displacement generator 300 and the reciprocating rotation part 500 may become complicated and unreliable.

Therefore, the moving hanger 100 may be configured such that one drive part 200 generates power to rotate multiple power transmitters 400.

Further, the moving hanger 100 may be configured such that the power generated by the drive part 200 is preferentially transmitted to some of the power transmitters 400 or some of the reciprocating rotation parts 500, and the remaining power transmitters 400 or the remaining reciprocating rotation parts 500 may receive the power secondarily.

For example, the reciprocating part 500 may be arranged to receive the power transmitted by the drive part 200 or the displacement generator 300 and transmit the power to some of the power transmitters 400. In other words, the moving hanger 100 is configured to intensively transmit the power generated by the drive part 200 to one reciprocating rotation part 500, such that the power transmission structure may be designed to be simple and the power loss may be minimized.

The moving hanger 100 may be arranged to transmit the power transmitted from the drive part 200 to a single reciprocating part 500 to rotate a specific power transmitter 400 connected to the reciprocating rotation part 500.

Further, the moving hanger 100 may further include a connector 600 arranged to transmit power delivered to a specific power transmitter 400 to the other power transmitters 400.

For example, the connector 600 may connect multiple power transmitter 400 to each other. Thus, when any one of the power transmitters 400 rotates, the connector 600 may cause all the multiple power transmitters 400 to rotate.

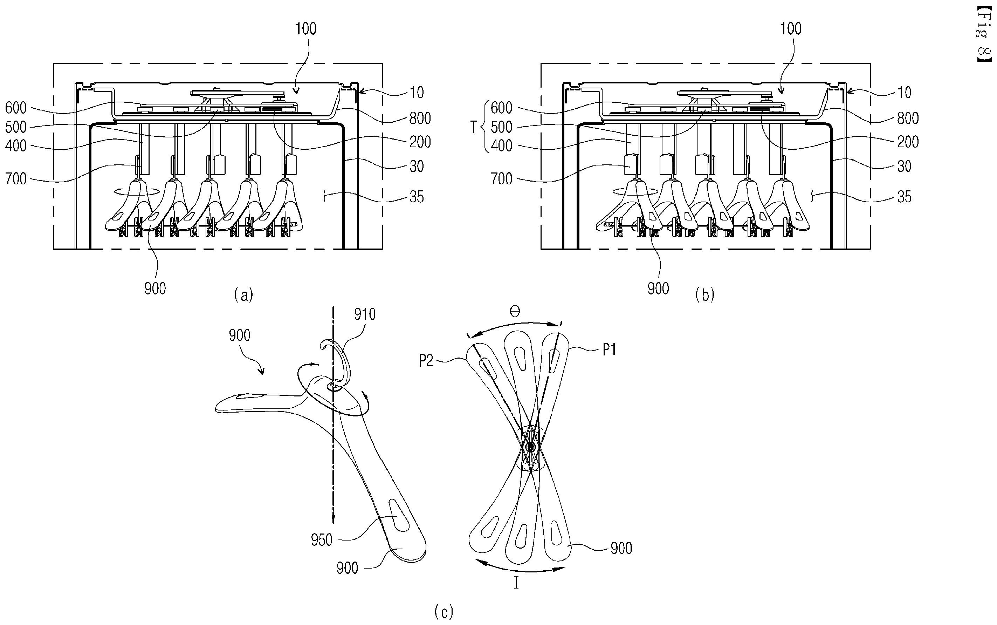

FIG. 8 illustrates the operation of the moving hanger according to the present disclosure.

Referring to FIG. 8-(a), when the drive part 200 is operated, the power transmitters 400 may be rotated to the right by the reciprocating part 500. At this time, all the power transmitters 400 connected to the connector 600 may be rotated to the right.

Referring to FIG. 8-(b), when the drive part 200 is further operated, the power transmitters 400 may be rotated to the left by the reciprocating part 500. At this time, all the power transmitters 400 connected to the connector 600 may be rotated to the left.

As this process is repeated, the power transmitters 400 may rotate left and right.

In this regard, the power transmitters 400 may be arranged to rotate left and right while being fixed at predetermined positions. The power transmitters 400 may be fixed to the support part 800 such that the positions of the power transmitters 400 do not change from front to back or side to side when rotated.

The power transmitters 400 may be fixed such that the positions thereof do not change with respect to the vertical direction, front-to-back direction, and width direction.

However, the power transmitter 400 may be arranged to rotate from side to side about the vertical direction or height direction in which the power transmitter extends as the axis of rotation. As a result, when the drive part 200 is operated, the hanging part 700 may be rotated from side to side about the power transmitter 400 as the axis in a reciprocating manner without any positional change.

Referring to FIG. 8-(c), the clothing hanger 900 may include a hook portion 910 mounted on the hanger part 700, and a seat portion 900 coupled to the hook portion 910. A surface of the seat portion 950 may be provided with a surface portion 950 that prevents the laundry from slipping.

The seat portion 950 may be symmetrically arranged around the hook portion 910. The clothing hanger 900 may be mounted on the hanging part 700 such that the seat portion 950 is disposed in a front-to-back direction.

When the power transmitter 400 rotates to the left, the left side of the seat portion 950 of the clothing hanger 900 may rotate to the left and the right side of the seat portion 950 may rotate to the right around the hook portion 910. In this case, the angle (I) by which the left side of the seat portion 950 rotates may be the same as the angle (theta) by which the right side of the seat portion 950 rotates, and the distance that the left side of the seat portion 950 moves may be the same as the distance that the right side of the seat portion 950 moves.

As a result, the weight and force moving to the left and the weight and force moving to the right with respect to the clothing hanger 900 may be equal to each other, and thus cancel each other.

Similarly, when the power transmitter 400 is rotated to the right, the weight and force moving to the left and the weight and force moving to the right with respect to the clothing hanger 900 may be equal to each other, and thus cancel each other.

As a result, when the power transmitter 400 rotates, the forces applied to the clothing hanger 900 may cancel each other, and thus the vibration force or inertia force generated by the clothing hanger 900 may be minimized. Thus, the inertia force or the like generated by the multiple power transmitters 400 may be minimized, thereby minimizing the vibration or noise occurring throughout the moving hanger 100 and drastically reducing the vibration or noise generated in the entire laundry treating apparatus 1.

As a result, even when the drive part 200 rotates at full power, vibration generated throughout the moving hanger 100 or the laundry treating apparatus 1 may not be significant.

Instead, each of the surfaces of the laundry mounted on the clothing hanger 900 may be shaken by rotating the hanger 900 from side to side, and therefore a large shaking force may be secured.

As described above, the power transmitter 400 may be arranged through the inner case 20 in a penetrating manner to receive the power and rotate clockwise and counterclockwise in a reciprocating manner. As a result, the power transmitter 400 may rotate from side to side in a reciprocating manner while remaining fixed in position in the upper portion of the inner case 20. The power transmitter 400 is fixed such that the position thereof does not vary in the up-down and left-right directions. Further, not only the upper portion of the power transmitter 400 but also the lower portion of the power transmitter 400 is fixed such that the position thereof does not vary in the up-down and left-right directions.

In other words, the power transmitter 400 may be arranged to rotate within a certain angle less than one revolution in a reciprocating manner with the center of rotation thereof fixed.

As a result, no matter how fast the power transmitter 400 rotates, the clothing hanger 900 remains fixed in position while one end thereof rotates or moves to one side and the other end thereof rotates or moves to the other side. Thus, the force and vibration transmitted to the power transmitter 400 may be canceled out.

Accordingly, the vibration and noise generated by the power transmitters 400, the hanging part 700, and the clothing hanger 900 inside the inner case 20 may be minimized.

Accordingly, the laundry treating apparatus of the present disclosure may rotate the drive part 200 at a higher rpm to cause the power transmitter 400 to reciprocate at a higher frequency. Thus, the laundry treating apparatus of the present disclosure may shake the laundry more vigorously.

For example, the conventional moving hanger M of the left and right reciprocating type and the moving hanger 100 of the present disclosure may be provided with a drive part 200 that exerts the same output power.

The conventional moving hanger M has a limitation that the drive part 200 cannot be operated above a specific rpm. If the drive part 200 is operated above the specific rpm, the vibration generated by the laundry may exceed a limit value, causing damage to the conventional moving hanger M, or causing excessive vibration or noise to the laundry treating apparatus.

On the other hand, the moving hanger 100 of the present disclosure is configured such that the power transmitter 400 reciprocates clockwise or counterclockwise in place. Therefore, as described above, no matter how fast the clothing hanger 900 rotates, the vibration or inertial force generated from the clothing hanger 900 and the laundry may be canceled out when transmitted to the power transmitter 400.

As a result, the moving hanger 100 of the present laundry treating apparatus may not be broken even when the drive part 200 is increased above a specific RPM, and vibration or noise that exceeds a limit value may be prevented from occurring in the laundry treating apparatus.

Therefore, the laundry treating apparatus of the present disclosure may drive the drive part 200 faster than a laundry treating apparatus using a conventional moving hanger, and may drive the power transmitter 400 at a higher frequency to shake the laundry more strongly.

Thus, the laundry treating apparatus according to the present disclosure may more reliably remove foreign matter stuck on the laundry with the moving hanger 100, and may more effectively remove wrinkles from the laundry.

Furthermore, the laundry treating apparatus according to the present disclosure may expose the laundry to more steam supplied by vibrating the laundry more rapidly.

Furthermore, the laundry treating apparatus according to the present disclosure may freely adjust the RPM of the drive part 200 to adapt the drive frequency or drive cycle of the power transmitter 400 to a course.

One embodiment of the moving hanger 100 according to the present disclosure may be arranged to transmit power from the drive part 200 to only one of the multiple power transmitters 400, and to transmit power transmitted to a specific power transmitter 400 to the remaining power transmitters 400 via the connector 600.

The displacement generator 300 or the reciprocating part 500 may be arranged to concentrate the power generated by one drive part 200 to one power transmitter 400. The connector 600 may transmit power transmitted to a specific power transmitter 400 to all power transmitters 400.

The connector 600 may be configured as a rigid body such that its length does not vary, and may be arranged to connect all the power transmitters 400.

Thus, all the power transmitters 400 may rotate in the same direction and at the same angle at once when the connector 600 is moved. As a result, the moving hanger 100 of the present disclosure may rotate multiple power transmitters 400 in a reciprocating manner simultaneously within the same angle with a single drive part 200.

The moving hanger 100 may include a drive part 200 fixed in an upper portion of the inner case 20 to provide power for moving the power transmitters, multiple reciprocating rotation parts 500 coupled to each of the multiple power transmitters 400 to receive power from the drive part 200 and rotate to repeatedly change the direction of rotation, and a connector 600 arranged to connect the multiple reciprocating rotation parts to each other.

The connector 600 may include a link bar arranged to connect the multiple reciprocating rotation part 500 to integrally rotate the multiple reciprocating rotation part 500 as a unit.

There may be a single connector 600 provided.

The connector 600 may be arranged to connect all the power transmitters 400.

However, when the connector 600 is arranged to connect the reciprocating rotation parts 500, the connector 600 may be installed above the support 800. Thereby, the connector 600 may be prevented from being exposed to the interior of the inner case 20.

The connector 600 may be coupled to one of the front or rear of the reciprocating rotation parts 500, and at least one of the displacement generator 300 and the drive part 200 may be disposed on the other of the front or rear of the reciprocating rotation parts 500. Thus, the connector 600 may be prevented from interfering with the drive part 200.

The connector 600 may be arranged to reciprocate in the width direction of the inner case 20 to rotate the multiple reciprocating rotation parts 500.

The drive part 200 may include a motor 210 arranged to rotate the rotation shaft 210, a power shaft 240 arranged to rotate when the rotation shaft 210 rotates, and a transmitter 230 connecting the power shaft 240 and the rotation shaft 210 to transmit a rotational force of the rotation shaft 210 to the power shaft 240.

The motor 210 may be fixed to the top of the inner case 20 to rotate the rotation shaft 220. The rotation shaft 220 may be configured to rotate in a reciprocating manner at a speed much faster than an appropriate cycle for rotating the power transmitter 400 by the motor 210. In view of this, reducing the RPM of the rotation shaft may result in a failure to transmit the output power of the motor 210 to the power transmitter 400.

To address this issue, the transmitter 230 may be arranged to transmit the output power of the rotation shaft 220 to the power transmitter 400 with the RPM of the rotation shaft 220 reduced.

The transmitter 230 may be coupled to the rotation shaft 220 to rotate, but may have a larger diameter than the rotation shaft 220. Thus, the transmitter 230 may transmit the torque of the rotation shaft 220 while rotating at a lower speed than the RPM of the rotation shaft 220.

The power shaft 240 may be rotated by the transmitter 230, may be provided separately from the rotation shaft 230, and may be arranged to transmit power directly to the power transmitter 400.

The reciprocating rotation part 500 may be coupled to the power transmitter 400 to rotate together with the power transmitter 400.

The reciprocating rotation part 500 may include a reciprocation lever 510 coupled to an upper portion of the power transmitter 400 to rotate the power transmitter 400.

The reciprocation lever 510 may be formed in the shape of a rib or rod with a center of rotation coupled to the support shaft 410.

The reciprocation levers 510 may be coupled to the upper ends of the power transmitters 400, respectively. Some of the reciprocation levers 510 may be coupled to the transmitter 230 to receive power from the motor 210.

The reciprocation levers 510 may be configured to rotate by a specific angle in a reciprocating manner when the transmitter 230 is rotated by the motor 210. The power transmitter 400 may be coupled to a center of rotation of the reciprocation lever 510 to rotate together with the reciprocation lever 510.

The multiple reciprocation levers 510 may be connected by the connector 600.

The connector 600 may be arranged to connect ends of the reciprocation levers 510.

Thereby, when any one of the reciprocation levers 510 rotates, the connector 600 may be moved to cause the multiple reciprocation levers 510 to rotate simultaneously and at once.

The power transmitters 400 and the reciprocation levers 510 may be supported on the support 800. The motor 210 and the transmitter 230 may also be supported on the support 800.

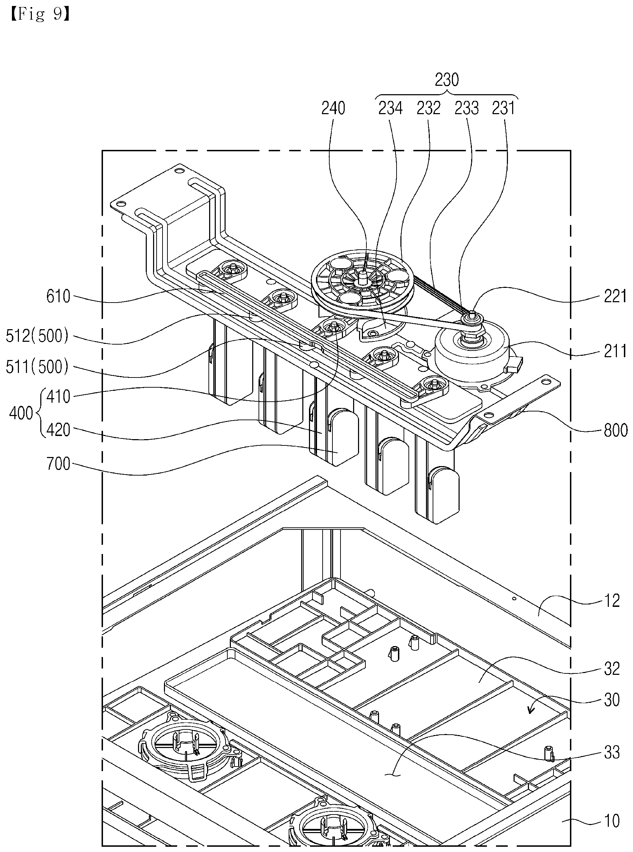

FIG. 9 illustrates the moving hanger 100 of the present disclosure separated from the inner case 20.

The power transmitter 400 may be arranged to extend from the top of the inner case downward, and the hanging part 700 may be coupled to the lower portion of the power transmitter 400.

The reciprocating rotation parts 500 may be coupled to the power transmitters 400, respectively. They may be coupled to upper portions of the power transmitters 400, and may thus be easily connected to the drive part 200.

The power transmitter 400 includes multiple power transmitters disposed at regular intervals along the width direction of the inner case, and the reciprocating rotation part 500 includes multiple reciprocating rotation parts disposed at regular intervals along the width direction of the inner case.

The connector 600 is arranged to connect the multiple the power transmitters 400 or the multiple the reciprocating parts 500 to each other. Thus, the connector 600 may be arranged to rotate all the power transmitters 400 or the multiple the reciprocating parts 500 simultaneously.

The power transmitter 400 may include a support shaft 410 coupled to the reciprocation lever 510 through the top of the inner case 20.

The support shaft 410 may extend the support 800 to be exposed to the space above the support 800 or on the inner case 20.

The power transmitter 400 may include an auxiliary support 420 coupled to the support shaft 410 and exposed to the accommodation space. The auxiliary support 420 may be rod-shaped and the hanging part 700 may be coupled and fixed to a lower portion thereof.

The auxiliary support 420 may be fixed to the support shaft 410 to rotate with the support shaft 410. Thus, when the support shaft 410 is rotated by the reciprocation lever 510, the auxiliary support 420 coupled to the support shaft 410 may also be rotated, causing the hanging part 700 to rotate from side to side.

The reciprocation lever 510 may include a main lever 511 arranged to receive power directly from the drive part 200 to rotate in a reciprocating manner, and an auxiliary lever 512 arranged to receive power from the main lever 511 via the connector 600.

The main lever 511 may be provided as a single element to receive power directly from the drive part 200.

In the drive part 200, the motor 210 may include a vertical motor 211 coupled to the support 800, and a vertical rotation shaft 221 rotated by the vertical motor 211.

The transmitter 230 may include a power pulley 231 coupled to the vertical rotation shaft 221 to rotate with the vertical rotation shaft 221, a transmission pulley 232 coupled to the power shaft 240 to rotate the power shaft 240, and a belt 233 connecting parts of the outer circumferential surfaces of the power pulley 231 and the transmission pulley 232.

The transmitter 230 may further include a pulley support 224 rotatably supporting the power shaft 240 and the transmission pulley 232. The pulley support 224 may be arranged to support the transmission pulley 232 to be disposed in parallel with the power pulley 231, and may be seated on the support 800.

The power shaft 240 may be arranged to transmit power transmitted from the rotation shaft 220 to one of the two ends of the main lever 511.

The displacement generator 300 may be coupled to the power shaft 240 to receive transmitted power. Further, the displacement generator 300 may be coupled to the main lever 511 to rotate the main lever 511 about the support shaft 410 in a reciprocating manner.

For example, the displacement generator 300 may include an eccentric shaft eccentrically coupled to the power shaft 240 to rotate a certain radius around the center of rotation of the power shaft 240.

The displacement generator 300 may be coupled to an end of the main lever 511. The displacement generator 300 may be rotated by the power shaft 240 to rotate the end of the main lever 511 around the support shaft 410 in a reciprocating manner.

The connector 600 may include a link bar 610 connecting one end of the main lever 511, which is not the end connected to the power shaft 240 or the displacement generator 300, and one end of the auxiliary lever 512.

The auxiliary lever 512 may be rotatably coupled to the remaining support shaft 410 that is not coupled to the main lever 511, and may extend in one direction from the portion coupled to the support shaft 410 to connect to the link bar 610.

The link bar 610 may be configured as a straight frame connecting one end of the main lever 511 and one end of each of the auxiliary levers 512. In this case, the one end of the main lever 511 and the one end of the auxiliary levers 512 may be disposed side by side with respect to the link bar 610 or the width direction.

The link bar 610 may be singular and arranged to rotate the main lever 511 and the auxiliary lever 512 simultaneously and at once around their respective support shafts 410.

The inner case 20 may be provided with through-holes 23 in which a portion of the support 800 is seated to expose the power transmitters 400 to the accommodation space 22.

The through-holes 23 may be provided in the top surface 22 of the inner case. The through-holes 23 may be arranged along a direction in which the power transmitters 400 are disposed.

For example, the power transmitters 400 may be arranged spaced apart from each other along a width direction of the inner case, and the through-holes 23 may be disposed along the width direction of the inner case.

The laundry treating apparatus 1 according to the present disclosure may further include a support frame 12 disposed on the outside of the inner case to support the cabinet 10.

The support frame 12 may be formed of a metallic material that is disposed at locations corresponding to corners of the cabinet 10 or corners of the inner case 20 to maintain the appearance of the laundry treating apparatus. The support 800 may be supported with both ends thereof seated on the support frame 12. Accordingly, unnecessary shock or load may be prevented from being transmitted to the top surface 22 of the inner case.

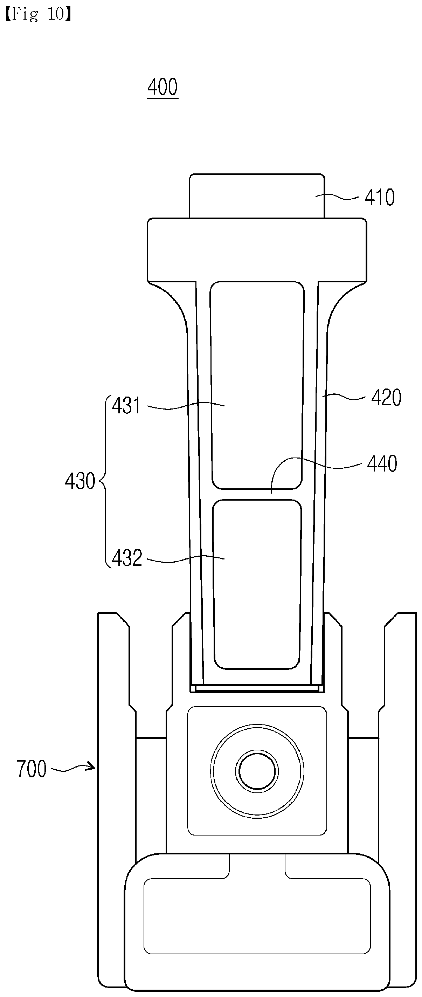

The moving hanger 100 may include the power transmitter 400, the drive part 200 configured to provide power to the power transmitter, and the hanging part 700 coupled to the power transmitter. FIG. 10 illustrates the hanging part 700 coupled to the power transmitter 400 among the elements of the moving hanger.

The power transmitter 400 may include a coupling portion 410 disposed at an upper end of the power transmitter 400, the drive part 200 being coupled to the coupling portion 410, a body 420 protruding downward from the coupling portion, and a hollow portion 430 formed inside the body and open on one side, and a reinforcing rib 440 disposed in the hollow portion to connect both side surfaces of the power transmitter and to divide the hollow portion into an upper and lower portion to reinforce the rigidity of the power transmitter.

The power transmitter 400 may be formed of plastic to minimize weight. Further, the power transmitter 400 may be manufactured by plastic injection molding to facilitate manufacturing. Thus, the hollow portion 430 may be provided because the mold must be withdrawn during manufacturing. The hollow portion may have space where a cooling fin is installed.

However, in injection molding, the plastic cannot be increased above a certain thickness to prevent appearance shrinkage. Accordingly, the thickness of the plastic may become thinner and the rigidity of the plastic may be lowered.

Therefore, a structure that may reinforce the rigidity of the plastic while securing space for installation of the cooling fin within a limited space may be required.

Accordingly, the power transmitter 400 may include the hollow portion 430 with one open side to facilitate injection molding, thereby securing space for installation of the cooling fin. In addition, the cooling fin and the mold may be discharged through the open side during injection molding.

5 Further, the hollow portion 430 may be provided with the reinforcing rib 440 to reinforce the rigidity that is weakened as a side effect of plastic injection molding.

Referring to FIG. 6, the conventional power transmitter is provided with the reinforcing rib in the form of a plate that divides the inside of the power transmitter into a front and rear portions, such that one surface of the reinforcing ribs faces forward in the power transmitter. The reinforcing rib having the shape as described above may support the load caused by gravity acting on the laundry by supporting the power transmitter in the height direction, but may not support the load caused by torsion due to rotation acting in the width direction of the power transmitter. On the other hand, the moving hanger of the present disclosure is arranged to rotate the laundry.

Therefore, in order to bear a load caused by torsion acting in the width direction, a single reinforcing rib 440 may be disposed to divide the hollow portion 430 into an upper hollow portion 431 and a lower hollow portion 432, as shown in FIG. 10. By disposing the reinforcing rib 440 to divide the hollow portion 430 into the upper and lower hollow portions, the power transmitter 400 may be supported in the width direction, thereby supporting the torsional load acting in the width direction during rotation.

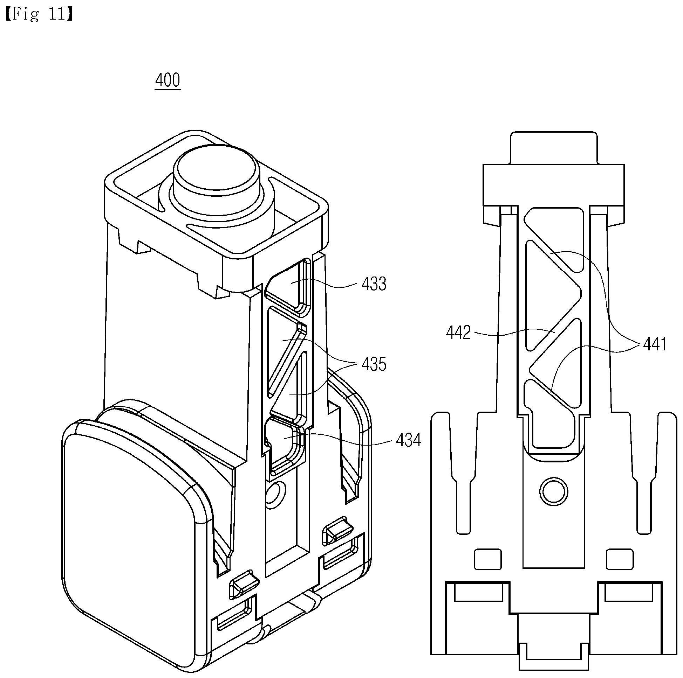

FIG. 11 illustrates one embodiment of the reinforcing rib 440.

The weight of the power transmitter 400 may be reduced by forming a deep hollow portion as shown in FIG. 11-(a), which may reduce the load on the top surface of the inner case 20 on which the moving hanger 100 is supported. Furthermore, by reducing the thickness of the plastic, shrinkage of the exterior may be prevented during injection molding, which may facilitate manufacturing and reduce material costs. For example, the depth of the hollow portion may be greater than half the length.

Alternatively, the hollow portion may be formed to have a shallow depth, as shown in FIG. 11(b), to provide strong support for the interior of the power transmitter 400 to further increase the rigidity of the power transmitter 400. For example, the depth of the hollow portion may be less than half the length.

The reinforcing rib 440 may include a first reinforcing rib 441 arranged such that one end thereof connected to one side surface of the power transmitter is located at a higher position than an opposite end thereof connected to an opposite side surface of the power transmitter. In other words, the first reinforcing rib 441 may be arranged to be inclined.

Further, the reinforcing rib 440 may include a second reinforcing rib 442 arranged such that one end thereof connected to the one side surface of the power transmitter is located at a lower position than an opposite end thereof connected to the opposite side surface of the power transmitter. In other words, the second reinforcing rib 442 may be arranged to be inclined in the opposite direction to the inclination of the first reinforcing rib.

Further, the reinforcing rib 440 may include a third reinforcing rib 443 arranged such that one end thereof connected to the one side surface of the power transmitter is located at a height corresponding to that of an opposite end thereof connected to the opposite side surface of the power transmitter. In other words, the third reinforcing rib may be parallel to the bottom surface.

The power transmitter 400 may include at least one of the first reinforcing rib 441, the second reinforcing rib 442, or the third reinforcing rib 443.

While FIG. 11 illustrates that three reinforcing ribs are provided, four or more reinforcing ribs may be provided. By increasing the number of the reinforcing ribs 440, stresses may be decentralized. That is, multiple first reinforcing ribs 441 may be arranged spaced apart from each other, multiple second reinforcing ribs 442 may be arranged spaced apart from each other, and multiple third reinforcing ribs 443 may be arranged spaced apart from each other.

For example, the power transmitter 400 may be provided with the first reinforcing ribs 441 and the second reinforcing ribs 442 arranged in an alternating manner. In this case, the first reinforcing ribs may be connected to the second reinforcing ribs. By arranging the first reinforcing ribs and the second reinforcing ribs to be connected, the stress may be decentralized.

In other words, the reinforcing rib 440 may be a truss structure.

Further, since each point at which the first reinforcing rib and the second reinforcing rib are connected in the hollow portion 430 is located at a different height, the reinforcing rib 440 connecting the respective points may provide support in the height direction as well, and thus may provide support for loads caused by torsion as well as loads caused by gravity as the hollow portion is divided into upper and lower portions.

Further, the second reinforcing rib 442 may not be arranged alternately with the first reinforcing rib 441, but may be arranged on at least one of the upper and lower sides of the multiple first reinforcing ribs. In other words, the number of the second reinforcing ribs may be less than the number of the first reinforcing ribs in the truss structure. As a result, the weight of the power transmitter 400 may be reduced.

The hollow portion 430 may include multiple hollow portions compartmentalized by the multiple reinforcing ribs 440.

That is, the hollow portion 430 may include a first hollow portion 433 positioned above the reinforcing rib disposed at the uppermost position among the multiple reinforcing ribs 440, a second hollow portion 434 disposed below the reinforcing rib disposed at the lowermost position among the multiple reinforcing ribs, and a third hollow portion 435 disposed between the first hollow portion and the second hollow portion. That is, the first hollow portion 433 may be located at the uppermost end and the second hollow portion 434 may be located at the bottom.

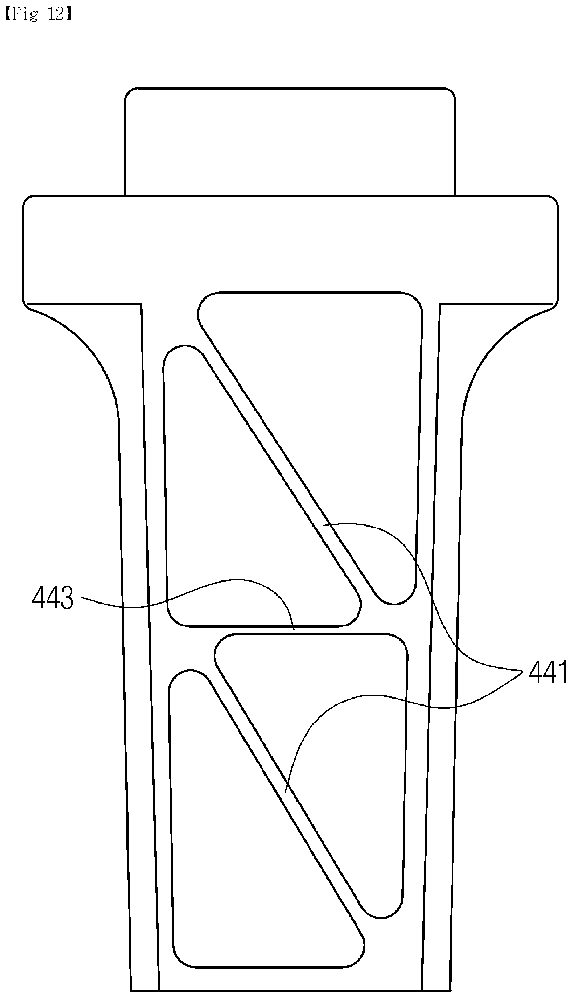

FIG. 12 illustrates an example of the reinforcing rib 440 including two first reinforcing ribs 441 and a third reinforcing rib arranged to connect the two first reinforcing ribs 441. However, embodiments are not limited thereto. The third reinforcing rib 443 may be arranged at at least one of a top and a bottom of at least one of the multiple first reinforcing ribs 441.

Stresses may be distributed at each point where the first reinforcing rib and the third reinforcing rib are connected.

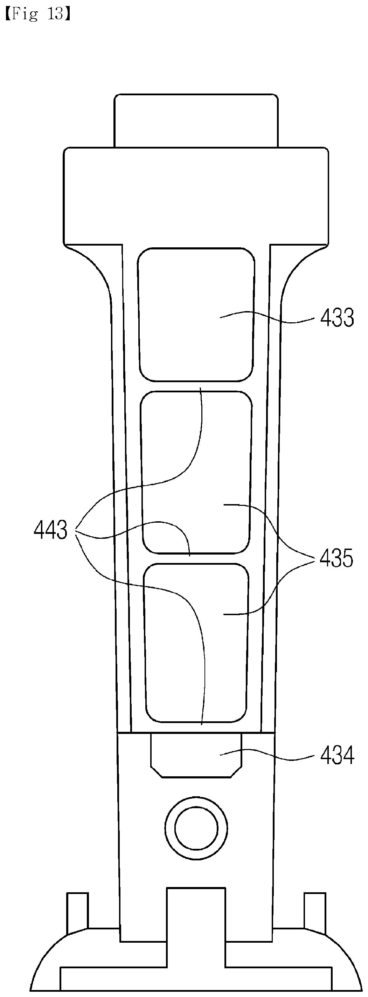

FIG. 13 illustrates an example of the reinforcing rib 440, wherein the third reinforcing ribs 443 are arranged spaced apart in the longitudinal direction of the power transmitter 440. FIG. 13 illustrates three third reinforcing ribs, but embodiments are not limited thereto. As the number of the reinforcing ribs increases, the support for torsional stresses may be improved.

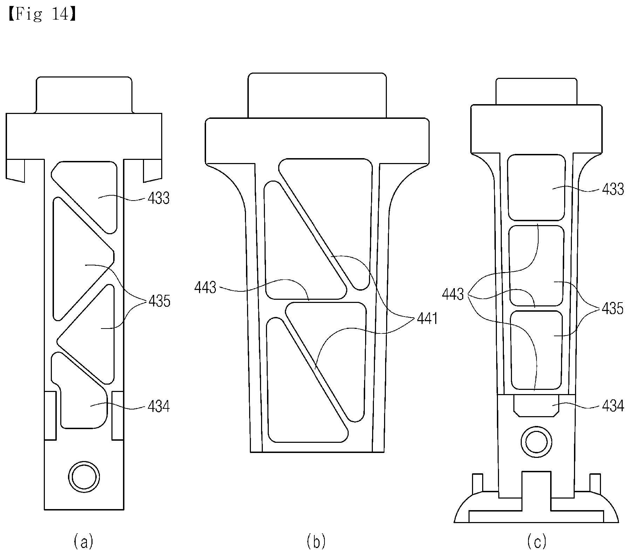

FIG. 14-(a) illustrates an alternating arrangement of the first reinforcing ribs 441 and the second reinforcing rib 442.

The hollow portion 430 may include a first hollow portion 433 disposed above the first reinforcing rib 441 at the uppermost position, a second hollow portion 434 disposed below the first reinforcing rib 441 at the lowermost position, and multiple third hollow portions 435 disposed between the first hollow portion and the second hollow portion.

Since the bottom of the power transmitter 400 supports the greatest load, the second hollow portions 435 may be the narrowest in width to support the load.

On the other hand, the top of the power transmitter 400 is subjected to the strongest external force from the drive part 200, and accordingly the first hollow portion 435 may be the narrowest width to support the load by the external force.

That is, the width of the third hollow portion 435 may be greater than the widths of the first hollow portion 433 and the second hollow portion 434.

The narrow width may mean that the distance between the point of action at which the load is applied and each point at which the reinforcing rib 440 connects to the power transmitter 400 is short, which may mean that the moment proportional to the distance from the point of action is small.

FIG. 14-(b) illustrates a third reinforcing rib 443 provided between multiple first reinforcing ribs 441.

The hollow portion 430 may have the third reinforcing rib 443 located below less half the length of the power transmitter 400 to reduce the width of the third hollow portion.

FIG. 14-(c) illustrates multiple third reinforcing ribs 443 spaced apart from each other in the longitudinal direction of the power transmitter 400.

The hollow portion 430 may include a first hollow portion 433 disposed above the third reinforcing rib 441 at the top, a second hollow portion 434 disposed below the third reinforcing rib 441 at the bottom, and multiple third hollow portions 435 disposed between the first hollow portion and the second hollow portion.

Since the bottom of the power transmitter 400 supports the greatest load, the second hollow portion 435 may have the narrowest width to support the load.

On the other hand, the top of the power transmitter 400 is subjected to the strongest external force from the drive part 200, and accordingly the first hollow portion 435 may have the narrowest width to support the load by the external force.

While FIG. 14 illustrates three reinforcing ribs 440 and four hollow portions 430, it is not excluded that the power transmitter 400 may include four or more reinforcing ribs and five or more hollow portions. Increasing the number of the reinforcing ribs may have the effect of dispersing stress.

It will be apparent to those skilled in the art that various modifications and variations can be made in the present disclosure without departing from the spirit and scope of the invention. Thus, it is intended that the present disclosure cover the modifications and variations of the present disclosure provided they come within the scope of the appended claims and their equivalents.

## Claims

1. A laundry treating apparatus comprising:
a cabinet;
an inner case disposed in the cabinet to provide an accommodation space to place laundry;
a machine chamber arranged to communicate with the inner case to supply at least one of steam or air to the accommodation space; and
a moving hanger arranged to shake the laundry placed in the accommodation space,
wherein the moving hanger comprises:
a power transmitter comprising a hollow portion disposed in the accommodation space in a height direction of the inner case and having at least a portion of one surface open;
a drive part coupled to the power transmitter to provide power to cause the power transmitter to rotate or translate; and
a hanging part provided on both side surfaces of the power transmitter except for the one surface of the power transmitter to place the laundry,
wherein the power transmitter comprises:
a reinforcing rib disposed to connect the both side surfaces of the power transmitter to divide the hollow portion into upper and lower portions to enhance rigidity of the power transmitter.

2. The laundry treating apparatus of claim 1, wherein the reinforcing ribs comprises at least one of:
a first reinforcing rib having one end connected to one of the side surfaces of the power transmitter and an opposite end connected to the other one of the side surfaces of the power transmitter, the one end being disposed at a higher position than the opposite end;
a second reinforcing rib having one end connected to the one side surface of the power transmitter and an opposite end connected to the other side surface of the power transmitter, the one end being disposed at a lower position than the opposite end; or
a third reinforcing rib having one end connected to the one side surface of the power transmitter and an opposite end connected to the other side surface of the power transmitter, the one end being disposed at a position corresponding to the other end.

3. The laundry treating apparatus of claim 2, wherein the first reinforcing rib comprises a plurality of first reinforcing ribs arranged spaced apart from each other.

4. The laundry treating apparatus of claim 2, wherein the second reinforcing rib comprises a plurality of second reinforcing ribs arranged spaced apart from each other.

5. The laundry treating apparatus of claim 2, wherein the third reinforcing rib comprises a plurality of third reinforcing ribs arranged spaced apart from each other.

6. The laundry treating apparatus of claim 3, wherein the second reinforcing rib is arranged on at least one of top and bottom of at least one of the first reinforcing ribs.

7. The laundry treating apparatus of claim 6, wherein the plurality of second reinforcing ribs and the plurality of first reinforcing ribs are alternately disposed in a longitudinal direction of the power transmitter.

8. The laundry treating apparatus of claim 3, wherein the third reinforcing rib is arranged on at least one of top and bottom of at least one of the first reinforcing ribs.

9. The laundry treating apparatus of claim 8, wherein the plurality of third reinforcing ribs and the plurality of first reinforcing ribs are alternately disposed in a longitudinal direction of the power transmitter.

10. The laundry treating apparatus of claim 3, wherein the third reinforcing rib is arranged on at least one of top and bottom of at least one of the second reinforcing ribs.

11. The laundry treating apparatus of claim 10, wherein the plurality of third reinforcing ribs and the plurality of second reinforcing ribs are alternately disposed in a longitudinal direction of the power transmitter.

12. The laundry treating apparatus of claim 1, wherein the hollow portion comprises:
a top hollow portion disposed above the reinforcing rib; and
a bottom hollow portion disposed below the reinforcing rib.

13. The laundry treating apparatus of claim 12, wherein the bottom hollow portion has a narrower width than the top hollow portion.

14. The laundry treating apparatus of claim 12, wherein the bottom hollow portion has a wider width than the top hollow portion.

15. The laundry treating apparatus of claim 12, wherein a width of the bottom hollow portion corresponds to a width of the top hollow portion.

16. The laundry treating apparatus of claim 1, wherein the reinforcing rib comprises a plurality of reinforcing ribs,
wherein the hollow portion comprises:
a first hollow portion disposed above a top reinforcing rib among the reinforcing ribs;
a second hollow portion disposed below a bottom reinforcing rib among the reinforcing ribs; and
a third hollow portion disposed between the first hollow portion and the second hollow portion.

17. The laundry treating apparatus of claim 16, wherein the second hollow portion has a narrowest width.

18. The laundry treating apparatus of claim 16, wherein the first hollow portion has a narrowest width.

19. The laundry treating apparatus of claim 16, wherein a width of the first hollow portion and a width of the second hollow portion are less than a width of the third hollow portion.

20. The laundry treating apparatus of claim 1, wherein the power transmitter comprises a plurality of power transmitters arranged spaced apart from each other.

21. The laundry treating apparatus of claim 1, wherein the machine chamber is disposed under the inner case.

22. The laundry treating apparatus of claim 1, wherein the power transmitter comprises:
a coupling portion coupled to the drive part; and
a body protruding downward from the coupling portion and containing the hollow portion,
wherein the hanging part is coupled to both side surfaces of the body.
